# EUROPEAN PATENT APPLICATION

(11) **EP 4 541 880 A1**
(43) Date of publication of application: **23.04.2025**
(21) Application number: 23823914.9
(22) Date of filing: 13.06.2023
(51) Int. Cl.: C12N 1/04, C12N 1/00, C12N 1/14, C12N 1/16, C12N 1/20, C12R 1/01, C12R 1/645

(54) **METHOD FOR USING PLASTIC FLUID TO STORE MICROORGANISM**

(30) Priority: 14.06.2022 JP 2022096055; 24.01.2023 JP 2023008889
(71) Applicant: Nissan Chemical Corporation, Tokyo 103-6119 (JP)
(72) Inventor: KANAKI, Tatsuro, Tokyo 103-6119 (JP); HATANAKA, Daisuke, Funabashi-shi, Chiba 274-0052 (JP); TAKAGI, Kimiko, Tokyo 123-0872 (JP)
(74) Representative: J A Kemp LLP
(86) International application number: PCT/JP2023/021854
(87) International publication number: WO 2023/243624

(57) **Abstract**

The present invention provides a plastic fluid composition containing the following:
(1) a microorganism
(2) a polysaccharide or a nanofiber composed of a polysaccharide,
wherein when the microorganism is a bacterium, it is comprised at a microbial density of 1×10⁷ CFU/mL or more, and is dispersed in the composition, and when the microorganism is a fungi, it is comprised at a microbial density of 1×10³ CFU/mL or more, and is dispersed in the composition.

## Description

### [Technical Field]

The present invention relates to the storage of microorganisms, and more specifically to a plastic fluid composition containing a microorganism and the like.

### [Background Art]

General techniques for storing microorganisms include subculture method, cryopreservation method, and freeze-drying method. In particular, the subculture method, which maintains microorganisms by seeding them in a solid gel or the like, is the most commonly used storage method. However, the subculture method has many problems, such as the great effort required to transplant microorganisms into a solid gel and the high risk of contamination by microorganisms other than the desired microorganism. The cryopreservation method is a technique for freezing microorganisms at extremely low temperatures using liquid nitrogen or the like, and is the most useful method for storing microorganisms for a long period of time. However, the cryopreservation method requires multiple steps of waking up microorganisms from a frozen state, such as thawing, seeding in a solid gel, and liquid culture, and is therefore not suitable for immediate use of microorganisms.

When considering not only storage but also subsequent use, the freeze-drying method is preferably used. This method is superior in the preservation properties of microorganisms, and when used, the freeze-dried powder of microorganisms can be used without being subjected to a waking up step or the like. However, the freeze-drying method also has problems. For example, when microorganisms present in a liquid are freeze-dried, the microorganisms are exposed to freezing stress and drying stress, which tends to reduce the survival rate and function of the microorganisms. In addition, it takes a certain amount of time for the freeze-dried microorganisms to return to their normal state and exert their physiological effects. Therefore, the freeze-dried microorganisms may be difficult to use under conditions where the processing time by the microorganisms is short. Furthermore, there are many species of microorganisms that are not suitable for freeze-drying.

In light of such background, the development of liquid-type microbial preparations that can store microorganisms for a relatively long period of time and can be used directly is underway. Liquid-type microbial preparations have advantages in terms of storage period and use form, but on the other hand, in liquid-type microbial preparations, the microorganisms in the preparation may settle during storage and form microbial aggregates at the bottom of the container. The microorganisms inside the aggregates cannot obtain oxygen or nutrients, and therefore, their survival rate or activity decreases. The sedimentation and aggregation of microorganisms can thus be one factor causing the deterioration of the quality of the microbial preparation. As a means of suppressing the occurrence of such settling and aggregation of microorganisms, a method of lowering the concentration of microorganisms in the preparation is adopted. Accordingly, most of the microbial preparations currently on the market have a low microbial concentration and are large in size. As a result, liquid-type microbial preparations have the problem of being expensive in terms of storage space, transportation, temperature control during storage, and the like. Furthermore, as mentioned above, liquid-type microbial preparations require stirring at regular intervals to maintain quality because sedimentation of microorganisms can occur, and large-sized liquid-type microbial preparations pose an issue in that stirring requires a great amount of effort.

To explain the stirring more specifically, some liquid-type microbial preparations are once fed to a culture device before use to grow microorganisms, and then used for the desired purpose. In the case of such microbial preparations, the microbial preparation is generally stored in a storage tank for liquid-type microbial preparations attached to the culture device, and only a part of the microbial preparation to be used is sent to the culture device, cultured, and used. In such embodiment, the liquid-type microbial preparation is stored in the storage tank for a certain period of time, but when the microorganisms contained in the preparation have a certain size, the microorganisms form sediments on the bottom of the tank. Therefore, in order to eliminate the bias of the microorganisms in the storage tank, it is necessary to stir the microbial preparation in the tank at all times or before use. Such stirring not only applies physical stress to the microorganisms, but also increases the risk of contamination by other microorganisms during stirring.

In addition, it is desirable to use sterile water as a solvent for liquid-type microbial preparations in order to prevent contamination with microorganisms other than the desired microorganism, but sterile water is relatively expensive. Therefore, use of non-sterile water (e.g., tap water) is desired. However, the problem of contamination with other microorganisms may also occur even when non-sterile water is used.

Furthermore, not only microbial preparations containing only one type of microorganism, but also preparations containing multiple types of microorganisms (e.g., bacteria and yeast) have been developed. It is difficult to apply the freeze-drying method to microbial preparations containing multiple microorganisms with different properties, and it is generally considered that maintaining them in the form of a liquid-type microbial preparation is preferable. However, even when microorganisms are stored in the form of a liquid-type microbial preparation, for example, whether the microorganisms form sediment or suspend in the solution depends on the characteristics of the microorganisms. As mentioned above, preparation of high quality liquid-type preparation containing multiple types of microorganisms is not easy because sedimentation of microorganisms can affect microbial viability.

Polysaccharides such as deacylated gellan gum (DAG) form a three-dimensional network (an amorphous structure) in a solution by aggregating through metal cations (e.g., divalent metal cations such as calcium ions). A technique has been reported in which cells are cultured in a liquid medium containing this three-dimensional network, whereby the cells are trapped in the three-dimensional network and do not form sediment, allowing the cells to be cultured in a suspended, uniformly dispersed state in the medium without the need for shaking or rotation operation (Patent Literature 1).

### [Citation List]

### [Patent Literature]

[Patent Literature 1]
WO 2014/017513

### [Summary of Invention]

### [Technical Problem]

In light of such background, a problem of the present invention is to provide a novel liquid-type microbial preparation that can solve multiple conventional problems at once.

### [Solution to Problem]

The present inventors have conducted intensive studies in an attempt to solve the above-mentioned problems and found that by suspending microorganisms in a plastic fluid,
(1) the suspension and dispersion state of the microorganisms can be maintained for a long period of time without a stirring operation, as a result of which the microorganisms can be stored with a high survival rate and the functional deterioration of the microorganisms can be suppressed;
(2) the microorganisms can be stored at a high density;
(3) contamination with microorganisms other than the desired microorganism can be suppressed;
(4) multiple types of microorganisms can be stored in good condition for a long period of time;
(5) the microorganisms can be used without any special treatment step when in use;
   and further,
(6) by storing the microorganisms using a plastic fluid, a decrease in the survival rate of the microorganisms can be suppressed even under high temperature or alkaline conditions. Based on such findings, they have conducted further studies and completed the present invention. Accordingly, the present invention provides the following.

[1] A plastic fluid composition comprising the following:
   (1) a microorganism
   (2) a polysaccharide or a nanofiber composed of a polysaccharide,
   wherein when the microorganism is a bacterium, it is comprised at a microbial density of 1×10⁷ CFU/mL or more, and is dispersed in the composition, and when the microorganism is a fungi, it is comprised at a microbial density of 1×10³ CFU/mL or more, and is dispersed in the composition.
[2] The composition of [1], wherein the composition has a yield value of 5 to 500 mPa and a viscosity of 1.5 to 200 mPa·s at 4 to 25°C.
[3] The composition of [1] or [2], comprising a polysaccharide.
[4] The composition of [3], wherein the polysaccharide is a deacylated gellan gum.
[5] The composition of [1] or [2], comprising a nanofiber composed of a polysaccharide.
[6] The composition of [5], wherein the nanofiber is a cellulose nanofiber.
[7] The composition of any of [1] to [6], which is for storing a microorganism.
[8] The composition of any of [1] to [7], comprising two types of microorganisms.
[9] The composition of [8], wherein the two types of microorganisms are a bacterium and a fungi.
[10] The composition of [9], wherein the bacterium is Burkholderia arboris and the fungi is Yarrowia lipolytica.
[11] A method for producing a microorganism-containing preparation, comprising dispersing a microorganism in a plastic fluid composition comprising a polysaccharide or a nanofiber composed of a polysaccharide,
   wherein when the microorganism is a bacterium, it is comprised at a microbial density of 1×10⁷ CFU/mL or more, and is dispersed in the composition, and when the microorganism is a fungi, it is comprised at a microbial density of 1×10³ CFU/mL or more, and is dispersed in the composition.
[12] The method of [11], wherein the plastic fluid has the following characteristics:
   having a yield value of 5 to 500 mPa and a viscosity of 1.5 to 200 mPa·s at 4 to 25°C.
[13] The method of [11] or [12], wherein the composition comprises a polysaccharide.
[14] The method of [13], wherein the polysaccharide is a deacylated gellan gum.
[15] The method of [11] or [12], wherein the composition comprises a nanofiber composed of a polysaccharide.
[16] The method of [15], wherein the nanofiber is a cellulose nanofiber.
[17] The method of any of [11] to [16], comprising two types of microorganisms.
[18] The method of [17], wherein the two types of microorganisms are a bacterium and a fungi.
[19] The method of [18], wherein the bacterium is Burkholderia arboris and the fungi is Yarrowia lipolytica.

### [Advantageous Effects of Invention]

According to the present invention, one type or multiple types of microorganisms can be stored in a liquid state at high concentrations for a long period of time without accompanying functional deterioration of the microorganisms, while suppressing contamination with other microorganisms. According to the present invention, moreover, a decrease in the survival rate of microorganisms can be suppressed even under high temperature conditions and alkali conditions.

### [Brief Description of Drawings]

[Fig. 1] Fig. 1 shows photographs of the suspension states of B. arboris and Y. lipolytica, after inoculation into a DAG-containing buffer solution-type plastic fluid/a DAG-free phosphate buffer and standing still for 16 days under refrigerated conditions. (Experimental Example 7)
[Fig. 2] Fig. 2 shows photographs of the suspension states of B. arboris and Y. lipolytica, after inoculation into a DAG-containing buffer solution-type plastic fluid/a DAG-free phosphate buffer and standing still for 31 days under refrigerated conditions. (Experimental Example 7)
[Fig. 3] Fig. 3 shows photographs of the suspension states of B. arboris and Y. lipolytica, after inoculation into a DAG-containing buffer solution-type plastic fluid/a DAG-free phosphate buffer and standing still for 90 days under refrigerated conditions. (Experimental Example 7)
[Fig. 4] Fig. 4 is a photograph showing the results of a growth test of B. arboris stored for 3.5 months in a DAG-containing buffer solution-type plastic fluid. B. arboris has a unique colony shape, with the colony surrounded by a translucent membrane. When a DAG-containing buffer solution-type plastic fluid was used, only B. arboris formed colonies, and colony formation of other microorganisms was not observed. (Experimental Example 11)
[Fig. 5] Fig. 5 is a photograph showing the results of a growth test of B. arboris stored for 3.5 months in a DAG-free phosphate buffer. When a DAG-free phosphate buffer was used, not only the colonies of B. arboris but also colony formation of other microorganisms was observed. (Experimental Example 11)
[Fig. 6] Fig. 6 is a photograph showing the status of contamination with other microorganisms when B. arboris and Y. lipolytica stored for about 1.5 months using a DAG-containing nutrient source aqueous solution-type plastic fluid/an aqueous solution not containing DAG but containing urea and various metal salts (microbial nutrient sources-containing aqueous solution) were re-cultured. (Experimental Example 12)
[Fig. 7] Fig. 7 shows photographs of time-course changes of B. arboris and Y. lipolytica that were stored using a DAG-containing nutrient source aqueous solution-type plastic fluid/a DAG-free microbial nutrient sources-containing aqueous solution. (Experimental Example 13)
[Fig. 8] Fig. 8 shows photographs of a dispersion state of microorganisms when the microorganisms were stored using a DAG-containing nutrient source aqueous solution-type plastic fluid or a CNF-containing nutrient source aqueous solution-type plastic fluid. (Experimental Example 14)

### [Description of Embodiments]

The present invention is described in detail in the following.

### 1. Plastic fluid composition

The present invention provides a plastic fluid composition containing (1) a microorganism and (2) a polysaccharide or a nanofiber composed of a polysaccharide, wherein when the microorganism is a bacterium, it is comprised at a microbial density of 1×10⁷ CFU/mL or more, and is dispersed in the composition, and when the microorganism is a fungi, it is comprised at a microbial density of 1×10³ CFU/mL or more, and is dispersed in the composition (hereinafter sometimes referred to as "the composition of the present invention").

In the present specification, the "plastic fluid" refers to a fluid that requires a yield stress in order to flow, i.e., a fluid having a yield value. The plastic fluid may be Bingham fluid or non-Bingham fluid.

In the composition of the present invention, the plastic fluid composition is not particularly limited as long as it is a plastic fluid that can retain microorganisms without sedimentation in a standing state. The yield value of the composition is preferably 5 mPa or more from the aspect of dispersing and retaining microorganisms, and preferably 500 mPa or less from the aspect of operability in, for example, filling storage containers. The yield value can be measured, for example, by the method described in the below-mentioned Examples. Specifically, it can be derived by measurement using a rheometer (manufactured by Anton Paar, model: MCR301, cone rotor: CP75-1).

The viscosity of the composition is preferably 1.5 to 200 mPa·s at 4 to 25°C, from the aspect of operability. The viscosity can be measured, for example, by the method described in the below-mentioned Examples. Specifically, it can be measured using an E-type viscometer (manufactured by Toki Sangyo Co., Ltd., TV-22 type viscometer, model: TVE-22L, cone rotor: standard rotor 1°34'×R24, rotation number 10 - 100 rpm).

A plastic fluid can be prepared by a method known per se. In one embodiment, a plastic fluid can be prepared by mixing a non-plastic fluid with polysaccharides.

Polysaccharides capable of turning non-plastic liquids into plastic fluids are known. As one example, polysaccharides wherein more than 10 single saccharides and oligosaccharides (e.g., triose, tetrose, pentose, hexsauce, heptose etc.) are polymerized, more preferably, acidic polysaccharides having an anionic functional group, can be mentioned. The acidic polysaccharide in the present specification is not particularly limited as long as it has an anionic functional group in the structure thereof, and includes, for example, polysaccharides having a uronic acid (e.g., glucuronic acid, iduronic acid, galacturonic acid, mannuronic acid), polysaccharides having a sulfuric acid group or phosphate group in a part of the structure thereof, and polysaccharides having the both structures. These polysaccharides may be naturally occurring polysaccharides, polysaccharides produced by microorganisms, polysaccharides produced by genetic engineering, or polysaccharides artificially synthesized using enzymes. More specifically, examples of acidic polysaccharide include those composed of one or more kinds selected from the group consisting of deacylated gellan gum (hereinafter sometimes to be referred to as "DAG"), gellan gum, rhamsan gum, diutan gum, hyaluronic acid, hexuronic acid, fucoidan, pectin, pectic acid, pectinic acid, heparan sulfate, heparin, heparitin sulfate, keratosulfate, chondroitin sulfate, dermatan sulfate, rhamnan sulfate, and salts thereof. Examples of the salt include, but are not limited to, alkali metal salts such as lithium, sodium, potassium, salts with alkaline earth metals such as calcium, barium, magnesium and salts with aluminum, zinc, steel, iron, ammonium, organic base and amino acid, and the like.

The polysaccharides used in the present invention is preferably DAG, diutan gum, hyaluronic acid, or a salt thereof, more preferably DAG.

The weight average molecular weight of these polysaccharides is preferably 10,000 to 50,000,000, more preferably 100,000 to 20,000,000, still more preferably 1,000,000 to 10,000,000. For example, the molecular weight can be measured based on pullulan by gel penetration chromatography (GPC). Furthermore, phosphorylated DAG can also be used. The phosphorylation can be performed by a known method.

Plural kinds (e.g., two kinds) of polysaccharides can be used in combination. The kind of the combination of the polysaccharides is not particularly limited, but the combination preferably contains DAG or a salt thereof. That is, a preferred combination of polysaccharides contains DAG or a salt thereof, and polysaccharides other than DAG and a salt thereof (e.g., xanthan gum, alginic acid, locust bean gum, methylcellulose, diutan gum, or a salt thereof). Examples of specific combination of polysaccharides include, but are not limited to, DAG and xanthan gum, DAG and sodium alginate, DAG and locust bean gum, DAG and methylcellulose, DAG and diutan gum, and the like.

The concentration of polysaccharide required to turn a non-plastic liquid into a plastic fluid depends on the type of polysaccharide. It may be set to generally 0.0005% to 1.0% (weight/volume), preferably 0.001% to 0.4% (weight/volume), more preferably 0.005% to 0.1% (weight/volume), further preferably 0.005% to 0.05% (weight/volume).

In the case of DAG, for example, it may be 0.001% to 1.0% (weight/volume), preferably 0.003% to 0.5% (weight/volume), more preferably 0.005% to 0.1% (weight/volume), further preferably 0.01% to 0.05% (weight/volume), most preferably 0.01% to 0.03% (weight/volume). In the case of native-type gellan gum, it may be 0.05% to 1.0% (weight/volume), preferably 0.05% to 0.1% (weight/volume).

When a combination of DAG or a salt thereof and polysaccharide other than DAG and a salt thereof is used, the concentration of DAG or a salt thereof is, for example, 0.005 - 0.02% (weight/volume), preferably 0.01 - 0.02% (weight/volume), and the concentration of polysaccharide other than DAG and a salt thereof is, for example, 0.005 - 0.4% (weight/volume), preferably 0.1 - 0.4% (weight/volume). Specific examples of the combination of the concentration range include the following.
DAG or a salt thereof: 0.005 - 0.08% (preferably 0.01 - 0.04%) (weight/volume)
polysaccharides other than DAG
xanthan gum: 0.01 - 0.2% (weight/volume)
sodium alginate: 0.01 - 0.2% (weight/volume)
locust bean gum: 0.01 - 0.2% (weight/volume)
methylcellulose: 0.01 - 0.2% (weight/volume) (preferably 0.2 - 0.4% (weight/volume))
diutan gum: 0.01 - 0.2% (weight/volume)
carboxymethylcellulose: 0.01 - 0.2% (weight/volume)

### [Metal cation]

In one embodiment, the composition of the present invention may be added with a metal cation, for example, a divalent metal cation (calcium ion, magnesium ion, zinc ion, iron ion, copper ion, etc.), preferably a calcium ion. In particular, when the nanofibers contained in the composition of the present invention are composed of a water-soluble polysaccharide such as deacylated gellan gum, the composition of the present invention preferably contains a monovalent and/or divalent metal cation(s). By containing a metal cation, the water-soluble polysaccharide such as deacylated gellan gum aggregates via the metal cation to form nanofibers in the composition, which then construct a three-dimensional network, resulting in the formation of nanofibers that can be cultured in a suspension state in the composition.

In another embodiment, nanofibers composed of polysaccharides may be used to prepare the plastic fluid.

By adding nanofibers composed of polysaccharides to a liquid that is not a plastic fluid, the liquid can be turned into a plastic fluid.

In the present specification, the nanofiber refers to a fiber having an average fiber diameter (D) of 0.001 to 1.00 µm. The average fiber diameter of the nanofiber to be used in the present invention is preferably 0.005 to 0.50 µm, more preferably 0.01 to 0.05 µm, further preferably 0.01 to 0.02 µm. When the average fiber diameter is less than 0.001 µm, the nanofibers may be too fine to obtain a sufficient flotation effect, due to which the properties of the composition containing them may not be improved.

The aspect ratio (L/D) of the nanofiber to be used in the present invention is obtained from average fiber length/average fiber diameter, and is generally 2 - 500, preferably 5 - 300, more preferably 10 - 250. When the aspect ratio is less than 2, there is a risk that the composition lacks dispersibility and the suspending action is not sufficiently obtained. When it exceeds 500, the fiber length is extremely large. By increasing the viscosity of the composition, the operability in filling or transferring the liquid composition into containers or feeding into culture tanks may be disrupted. In addition, the composition becomes less transparent to visible light, which decreases transparency, and observation of the microorganisms over time may become difficult.

For example, the average fiber diameter (D) of the nanofiber can be determined as follows. First, a hydrophilizing treatment of a collodion support film manufactured by Okenshoji Co., Ltd. was performed for 3 min by an ion cleaner (JIC-410) manufactured by JEOL Ltd., several drops of a nanofiber dispersion (diluted with ultrapure water) to be the evaluation target was added dropwise, and dried at room temperature. This was observed under a transmission electron microscope (TEM, H-8000) (10,000-fold) manufactured by Hitachi, Ltd. at an accelerating voltage 200 kV. Using the obtained image, the fiber diameter of each one of the nanofibers (specimen number: 200 - 250) was measured, and the mean thereof was taken as the average fiber diameter (D).

In addition, the average fiber length (L) was determined as follows. A nanofiber dispersion to be the evaluation target is diluted to 100 ppm with pure water, and nanofibers are uniformly dispersed using an ultrasonic cleaner. The nanofiber dispersion is cast on a silicon wafer subjected in advance to a hydrophilizing treatment of the surface with conc. sulfuric acid, dried at 110°C for 1 hr and used as a sample. Using an image obtained by observing the obtained sample under a scanning electron microscope (SEM, JSM-7400F) (2,000-fold), the fiber length of each one of the nanofibers (specimen number: 150 - 250) is measured, and the mean thereof is taken as the average fiber length (L).

The nanofiber used in the present invention is, upon mixing with a liquid that is not a plastic fluid, uniformly dispersed in the liquid while maintaining the primary fiber diameter, substantially retains the cell and/or tissue without substantially increasing the viscosity of the liquid, and shows an effect of preventing sediment thereof.

The viscosity of the liquid containing the nanofiber can be measured using a tuning fork vibration type viscometer (SV-1A, A&D Company Ltd.) under 25°C conditions.

Polysaccharides that can be used as starting materials for nanofibers include not only naturally occurring polysaccharides, but also substances produced by microorganisms, substances produced by genetic engineering, or substances artificially synthesized using enzymes or chemical reactions. The polysaccharides that constitute the nanofibers used in the present invention are preferably naturally occurring substances (i.e., substances extracted from nature) or substances obtained by modifying them by chemical reactions or enzymatic reactions.

In one embodiment of the present invention, examples of the polysaccharides constituting the nanofiber include, but are not limited to, celluloses such as cellulose, hemicellulose and the like.

Cellulose is a natural polymer compound wherein D-glucopyranoses which are a 6-membered ring of glucose, are β-1, 4 glucoside bonded. As the starting material, for example, plant-derived cellulose such as lumber, bamboo, hemp, jute, kenaf, cotton, agricultural crops and food residue and the like, or cellulose of microorganism production or animal production such as bacterial cellulose, Cladophora, Glaucocystis, Valonia, Tunicate cellulose and the like can be used. In the plant-derived celluloses, very fine fibers called microfibrils are bundled to form higher structures in stages such as fibril, lamella and fibre cell. In addition, in bacterial cellulose, cellulose microfibrils secreted from bacterial cells and having the unchanged thickness form a fine net structure.

In the present invention, cellulose material having high purity such as cotton, bacterial cellulose and the like can be directly used. However, other plant-derived cellulose and the like are preferably used after isolation and purification. Cellulose preferably used in the present invention includes cotton cellulose, bacterial cellulose, kraft pulp cellulose, crystalline cellulose and the like. In particular, kraft pulp cellulose can be preferably used because of its high suspending action.

Nanofibers composed of polysaccharides can be prepared by a method known per se.

In one embodiment, in the case of cellulose nanofiber, the nanofiber is generally obtained by pulverizing the starting material. While the pulverization method is not limited, a method affording a strong shear force such as a medium stirring mill, for example, a high-pressure homogenizer, a grinder (stone mill), a bead mill and the like is preferable for subdivision to the below-mentioned fiber diameter and fiber length meeting the object of the present invention.

Among these, subdivision by a high-pressure homogenizer is preferable, and, for example, subdivision (pulverization) by the wet grinding method disclosed in JP-A-2005-270891 or JP-B-5232976 is desirable. Specifically, the starting material is pulverized by spraying a dispersion of a starting material from a pair of nozzles at a high-pressure and bombarding each other, and, for example, Star Burst system (high-pressure pulverization device manufactured by Sugino Machine Limited) or NanoVater (high-pressure pulverization device of YOSHIDA KIKAI CO., LTD.) is used therefor.

In the subdivision (pulverization) of a starting material by the aforementioned high-pressure homogenizer, the degree of subdivision and homogenization depends on the pressure in pumping into an ultrahigh-pressure chamber in a high-pressure homogenizer, and the number (treatment number) of passage through the ultrahigh-pressure chamber, and the concentration of the starting material in the aqueous dispersion. The pumping pressure (treatment pressure) is generally 50 - 250 MPa, preferably 150 - 245 MPa. When the pumping pressure is less than 50 MPa, subdivision of nanofiber may be insufficient and the effect expected to be afforded by the subdivision may not be achieved.

The concentration of the starting material in an aqueous dispersion during the subdividing treatment is 0.1 mass % - 30 mass %, preferably 1 mass % - 10 mass %. When the concentration of the starting material in an aqueous dispersion is less than 0.1% by mass, the producibility becomes low, and when the concentration is higher than 30% by mass, pulverization efficiency becomes low and the desired nanofiber cannot be obtained. While the number of subdivision (pulverization) treatments is not particularly limited, it varies depending on the concentration of the starting material in the aforementioned aqueous dispersion. When the concentration of the starting material is 0.1 - 1 mass %, 10 to 100 times of treatments is sufficient for pulverization, but when it is 1 - 10 mass %, about 10 to 1000 times of treatments are required. A high concentration exceeding 30% by mass is unrealistic from an industrial point of view because several thousand times of treatments are necessary and the viscosity increases to the point where handling is hindered.

The concentration of nanofibers in the composition of the present invention is not particularly limited as long as it is a concentration that can turn a liquid that is not a plastic fluid into a plastic fluid. It is generally 0.0001% to 1.0% (weight/volume), for example, 0.0005% to 1.0% (weight/volume), preferably 0.001% to 0.5% (weight/volume), more preferably 0.005% to 0.1% (weight/volume), further preferably 0.005% to 0.05% (weight/volume).

For example, in the case of cellulose nanofibers, it is generally 0.0001% to 1.0% (weight/volume), for example, 0.0005% to 1.0% (weight/volume), preferably 0.001% to 0.5% (weight/volume), more preferably 0.01% to 0.1% (weight/volume), further preferably 0.01% to 0.05% (weight/volume).

In the case of pulp cellulose nanofibers among cellulose nanofibers, the lower limit of the concentration is preferably 0.01% (weight/volume) or more, 0.015% (weight/volume) or more, 0.02% (weight/volume) or more, 0.025% (weight/volume) or more, or 0.03% (weight/volume) or more. In the case of pulp cellulose nanofiber, the upper limit of the concentration is preferably 0.1% (weight/volume) or less, or 0.04% (weight/volume) or less.

In the case of microcrystalline cellulose nanofiber, the lower limit of the concentration is preferably 0.01% (weight/volume) or more, 0.03% (weight/volume) or more, or 0.05% (weight/volume) or more. In another embodiment, the lower limit of the concentration of the microcrystalline cellulose nanofiber is preferably 0.03% (weight/volume) or more, or 0.05% (weight/volume) or more. In the case of microcrystalline cellulose nanofibers, the upper limit of the concentration is preferably 0.1% (weight/volume) or less.

Water-insoluble nanofibers such as cellulose nanofibers generally do not substantially increase the viscosity of the composition as long as the concentration is 0.1% (weight/volume) or less.

The concentration of nanofibers in the composition can be calculated by the following formula. concentration (%) (weight/volume)=nanofiber weight (g)/plastic fluid composition volume (ml)×100

When preparing the composition of the present invention, a plastic fluid is prepared by adding nanofibers composed of polysaccharides to a liquid that is not a plastic fluid. The liquid that is not a plastic fluid to which polysaccharides or nanofibers composed of polysaccharides are added is not particularly limited as long as it does not affect the survival of microorganisms. Examples of the liquid that is not a plastic fluid include, but are not limited to, water, buffer, and liquid medium for culturing microorganisms. In one preferred embodiment, the liquid that is not a plastic fluid may be a buffer.

The composition of the present invention contains microorganisms.

The microorganisms to be contained are not particularly limited, and examples thereof include bacteria and fungi.

The bacteria to be contained in the composition of the present invention are not particularly limited, and as one example, bacteria capable of decomposing fats and oils are preferred. Examples of the bacteria capable of decomposing fats and oils include, but are not limited to, Bacillus bacteria, Corynebacterium bacteria, Rhodococcus bacteria, Burkholderia bacteria, Acinetobacter bacteria, Pseudomonas bacteria, Alcaligenes bacteria, Rhodobacter bacteria, Ralstonia bacteria, Acidovorax bacteria, Serratia bacteria, Flavobacterium bacteria, Lactobacillus bacteria, Enterococcus bacteria, Sphingomonas bacteria, Staphylococcus bacteria, Rhizobium bacteria, Tetrasphaera bacteria, Stenotrophomonas bacteria, and the like. In one embodiment, Burkholderia bacteria are preferred, and Burkholderia arboris is preferred.

The fungi to be contained in the composition of the present invention are not particularly limited, and as one example, fungi capable of decomposing fats and oils are preferred. Examples of the fungi capable of decomposing fats and oils include, but are not limited to, Yarrowia fungi, Cryptococcus fungi, Trichosporon fungi, and Hansenula fungi, Candida fungi, Pichia fungi, Saccharomyces fungi, Kluyveromyces fungi, Aspergillus fungi, Penicillium fungi, Rhizopus fungi, Fusarium fungi, Humicola fungi, and the like. In one embodiment, Yarrowia fungi are preferred, and Yarrowia lipolytica is preferred.

The composition of the present invention may contain one type of microorganism, or two or more types of microorganisms.

In one embodiment of the composition of the present invention, the composition of the present invention may contain two types of microorganisms. The two types of microorganisms may be any of a bacterium and a bacterium, a fungus and a fungus, and a bacterium and a fungus. Preferably, the two types of microorganisms may be a bacterium and a fungus. More preferably, the bacterium may be a Burkholderia bacterium, and the fungus may be a Yarrowia fungus. In another embodiment, the bacterium may be Burkholderia arboris (B. arboris), and the fungus may be Yarrowia lipolytica (Y. lipolytica).

In the composition of the present invention, microorganisms are contained at a high density. As the microorganism contained in the composition of the present invention, bacterium and/or fungi can be mentioned.

When the microorganism contained in the composition of the present invention is a bacterium, the microbial density of the bacterium is generally 1×10⁷ CFU/mL or more, preferably 2×10⁷ CFU/mL or more, 3×10⁷ CFU/mL or more, 4×10⁷ CFU/mL or more, more preferably 5×10⁷ CFU/mL or more, 6×10⁷ CFU/mL or more, 7×10⁷ CFU/mL or more, 8×10⁷ CFU/mL or more, 9×10⁷ CFU/mL or more, further preferably 1×10⁸ CFU/mL or more.

The upper limit of the microbial density of the bacterium is not particularly limited as long as it does not adversely affect the survival of the bacterium. It is generally 1×10¹¹ CFU/mL or less, preferably 9×10¹⁰ CFU/mL or less, 8×10¹⁰ CFU/mL or less, 7×10¹⁰ CFU/mL or less, 6×10¹⁰ CFU/mL or less, more preferably 5×10¹⁰ CFU/mL or less, 4×10¹⁰ CFU/mL or less, 3×10¹⁰ CFU/mL or less, 2×10¹⁰ CFU/mL or less, particularly preferably 1×10¹⁰ CFU/mL or less.

When the microorganism contained in the composition of the present invention is a fungus, the microbial density of the fungus is generally 1×10³ CFU/mL or more, preferably 2×10³ CFU/mL or more, 3×10³ CFU/mL or more, 4×10³ CFU/mL or more, more preferably 5×10³ CFU/mL or more, 6×10³ CFU/mL or more, 7×10³ CFU/mL or more, 8×10³ CFU/mL or more, 9×10³ CFU/mL or more, further preferably 1×10⁴ CFU/mL or more.

The upper limit of the microbial density of the fungus is not particularly limited as long as it does not adversely affect the survival of the fungus. It is generally 1×10⁹ CFU/mL or less, preferably 9×10⁸ CFU/mL or less, 8×10⁸ CFU/mL or less, 7×10⁸ CFU/mL or less, 6×10⁸ CFU/mL or less, more preferably 5×10⁸ CFU/mL or less, 4×10⁸ CFU/mL or less, 3×10⁸ CFU/mL or less, 2×10⁸ CFU/mL or less, further preferably 1×10⁸ CFU/mL or less.

When the composition of the present invention contains both bacteria and fungi, the bacteria and fungi may each be independently contained at the aforementioned microbial densities.

When the composition of the present invention contains two types of bacteria or two types of fungi, each bacterium/fungus may each be independently contained at the aforementioned microbial densities.

Since the composition of the present invention has the physical properties of a plastic fluid, the microorganisms can be maintained in a dispersion state in the composition for a long period of time without sedimentation on the bottom surface of a container even without stirring or shaking. Therefore, in one embodiment, the composition of the present invention can be preferably used as a composition for storing microorganisms.

The period during which the composition of the present invention can disperse microorganisms without stirring or the like may be generally one day or more, preferably 7 days or more, 14 days or more, 21 days or more, 28 days or more, 35 days or more, 42 days or more, 49 days or more, 56 days or more, 63 days or more, 70 days or more, 77 days or more, 84 days or more, 91 days or more, 98 days or more, 105 days or more, 112 days or more, 119 days or more, 126 days or more, 133 days or more, 140 days or more, 147 days or more, 154 days or more, 161 days or more, 168 days or more, 175 days or more, 182 days or more, more preferably 1 year or more.

In one embodiment, the composition of the present invention may contain components other than the microorganisms and polysaccharides or nanofibers composed of polysaccharides. Examples of such component include substances that serve as nutrient sources for microorganisms or that can enhance the biological functions and survival of microorganisms.

The substances that serve as nutrient sources for microorganisms are not particularly limited as long as they can be the nutrient sources for microorganisms. For example, carbon source, nitrogen source, inorganic salt and the like can be mentioned.

Examples of the carbon source include sugars such as glucose, fructose, cellobiose, raffinose, xylose, maltose, galactose, sorbose, glucosamine, ribose, arabinose, rhamnose, sucrose, trehalose, α-methyl-D-glucoside, salicin, melibiose, lactose, melezitose, inulin, erythritol, glucitol, mannitol, galactitol, N-acetyl-D-glucosamine, starch, starch hydrolysates, molasses, blackstrap molasses, and the like, natural products such as wheat and rice, alcohols such as glycerol, methanol, and ethanol, organic acids such as acetic acid, lactic acid, succinic acid, gluconic acid, pyruvic acid, and citric acid, and hydrocarbons such as hexadecan. One or more kinds of the above-mentioned carbon sources may be contained.

Examples of the nitrogen source include organic nitrogen sources such as meat extract, fish meat extract, peptone, polypeptone, yeast extract, malt extract, soybean hydrolysate, soybean powder, casein, milk casein, casamino acid, various amino acids such as glycine, glutamic acid, aspartic acid, corn steep liquor, other animal, plant, and microbial hydrolysates, and inorganic nitrogen sources such as ammonia, ammonium salts such as ammonium nitrate, ammonium sulfate, and ammonium chloride, nitrates such as sodium nitrate, nitrites such as sodium nitrite, and urea. One or more kinds of the above-mentioned nitrogen sources may be contained.

Examples of inorganic salt include salts of magnesium, manganese, calcium, sodium, potassium, copper, iron, zinc, etc. (e.g., phosphates, hydrochlorides, sulfates, acetates, carbonates, bicarbonates, chlorides, etc.). One or more kinds of the above-mentioned inorganic salts may be contained.

The substances that can enhance the biological functions and survival of microorganisms are not particularly limited as long as they can achieve the desired purposes, and may be selected as appropriate according to the type of microorganism. For example, when the microorganism is capable of decomposing fats and oils, oil may be added to maintain or enhance the biological function thereof.

In the present specification, the "oil" refers to edible or industrial fats and oils that are rich in glycerides such as triglycerides, diglycerides, and monoglycerides, as well as fatty acids. Examples of the oil to be added to the composition of the present invention include edible fats and oils such as olive oil, canola oil, coconut oil, sesame oil, rice oil, rice bran oil, safflower oil, soybean oil, corn oil, rapeseed oil, palm oil, palm kernel oil, sunflower oil, cottonseed oil, coconut oil, peanut oil, beef tallow, lard, chicken oil, fish oil, whale oil, butter, margarine, fat spread, and shortening; industrial fats and oils such as linseed oil, jatropha oil, tall oil, crambe oil, castor oil, and jojoba oil; and fatty acids such as butyric acid, hexanoic acid, heptanoic acid, octanoic acid, decanoic acid, lauric acid, tridecanoic acid, myristic acid, pentadecanoic acid, palmitic acid, heptadecanoic acid, stearic acid, arachidic acid, behenic acid, lignoceric acid, decenoic acid, myristoleic acid, pentadecenoic acid, palmitoleic acid, heptadecenoic acid, oleic acid, icosenoic acid, docosenoic acid, tetracosenoic acid, hexadecadienoic acid, hexadecatrienoic acid, hexadecatetraenoic acid, linoleic acid, α-linolenic acid, γ-linolenic acid, octadecatetraenoic acid, icosadienoic acid, icosatrienoic acid, icosatetraenoic acid, arachidonic acid, icosapentaenoic acid, henicosapentaenoic acid, docosadienoic acid, docosatetraenoic acid, docosapentaenoic acid, and docosahexaenoic acid are preferred.

The amount of oil to be added is not particularly limited and may be selected as appropriate according to the type of microorganism. In one example, the oil may be added in, but not limited to, an amount of 1 to 30 g, more preferably 5 to 15 g, per 1 L of the composition of the present invention. The oil may be added alone or in combination of two or more kinds thereof.

Metal salts may also be added to the composition of the present invention. Examples of the metal salts include, but are not limited to, sulfates, sulfites, hyposulfites, persulfates, thiosulfates, carbonates, phosphates, pyrophosphates, hydrochlorides, nitrates, nitrites, acetates, propionates, butyrates, citrates, oxalates, and halides (e.g., fluorides, chlorides, bromides, and iodides) of metal elements such as sodium, potassium, magnesium, calcium, manganese, iron, nickel, zinc, and copper. More specifically, examples of metal salts include sodium sulfate, sodium sulfite, sodium hyposulfite, sodium thiosulfate, sodium carbonate, sodium persulfate, monosodium phosphate, disodium phosphate, trisodium phosphate, sodium acetate, sodium nitrate, sodium nitrite, sodium citrate, sodium oxalate, sodium chloride, potassium sulfate, potassium sulfite, potassium hyposulfite, potassium thiosulfate, potassium carbonate, potassium persulfate, monopotassium phosphate, dipotassium phosphate, tripotassium phosphate, potassium acetate, potassium nitrate, potassium nitrite, potassium citrate, potassium oxalate, potassium chloride, magnesium sulfate, magnesium sulfite, magnesium thiosulfate, magnesium carbonate, magnesium monophosphate, magnesium diphosphate, magnesium triphosphate, magnesium pyrophosphate, magnesium nitrate, magnesium nitrite, magnesium acetate, magnesium citrate, magnesium oxalate, magnesium chloride, calcium sulfate, calcium sulfite, calcium thiosulfate, calcium carbonate, calcium nitrate, calcium nitrite, calcium acetate, calcium citrate, calcium oxalate, and calcium chloride. The metal salt may be added alone or in combination of two or more kinds thereof.

In one embodiment, from the aspect of good survival rate of microorganisms during storage, the metal salt is preferably a sulfate, carbonate, phosphate, or a combination thereof of an element selected from the group consisting of sodium, potassium, magnesium, and calcium, and more preferably a sulfate of magnesium and/or calcium. The "metal salt" in the present specification also includes hydrates and solvates of metal salts.

In another embodiment, the composition of the present invention may contain monosaccharides such as glucose and fructose; disaccharides other than trehalose, such as sucrose, lactose and maltose; polysaccharides such as cyclodextrin, hydroxypropyl cellulose, hydroxypropylmethyl cellulose, carboxymethyl cellulose, crystalline cellulose and corn starch; proteins such as soy protein; protein hydrolysates and peptides such as soy peptides, gelatin, peptone and tryptone; fats and oils such as soybean oil, rapeseed oil, palm oil, sesame oil and olive oil; vitamins such as ascorbic acid or a salt thereof and tocopherol; surfactants such as polyglycerin fatty acid esters, sucrose fatty acid esters and sorbitan fatty acid esters; polyethylene glycol, glycerin, and the like.

The composition of the present invention may also contain a pH adjuster and the like.

### 2. Production method of microbial preparation

The present invention also provides a method for producing a microorganism-containing preparation, comprising dispersing a microorganism in a plastic fluid composition comprising a polysaccharide or a nanofiber composed of a polysaccharide, wherein when the microorganism is a bacterium, it is comprised at a microbial density of 1×10⁷ CFU/mL or more, and is dispersed in the composition, and when the microorganism is a fungi, it is comprised at a microbial density of 1×10³ CFU/mL or more, and is dispersed in the composition (hereinafter sometimes referred to as "the production method of the present invention").

The plastic fluid composition, polysaccharides, nanofibers composed of polysaccharides, microorganism and concentration thereof and the like in the production method of the present invention are the same as those explained in the composition of the present invention.

The present invention is explained in more detail by referring to Examples below. However, the present invention is not limited at all by the Examples.

### [Example]

### [Experimental Example 1] Production of deacylated gellan gum (DAG) aqueous solution

A 0.8% (weight/volume) DAG aqueous solution was prepared by adding 100 parts by volume of tap water and 0.8 parts by weight of deacylated gellan gum (hereinafter sometimes referred to as "DAG"; manufactured by Sansho Co., Ltd.) to a glass culture solution bottle and sterilizing the bottle in an autoclave (121°C, 20 min).

### [Experimental Example 2] Production of plastic fluid containing deacylated gellan gum

A 2x concentrated phosphate buffer was prepared by dissolving potassium dihydrogen phosphate (KH₂PO₄) and disodium hydrogen phosphate (Na₂HPO₄) in tap water at a ratio of KH₂PO₄:6 g/L, Na₂HPO₄:4 g/L, or a 2x concentrated phosphate buffer was prepared by dissolving potassium dihydrogen phosphate (KH₂PO₄) and disodium hydrogen phosphate (Na₂HPO₄) in a ratio of KH₂PO₄:12 g/L, Na₂HPO₄:8 g/L. In addition, an aqueous solution containing tap water, urea, and various metal salts was prepared as an aqueous solution containing nutrient sources such as carbon sources, nitrogen sources, and inorganic salts for microorganisms having an ability to decompose oil (hereinafter sometimes referred to as "microbial nutrient sources-containing aqueous solution"). The microbial nutrient sources-containing aqueous solution is a non-plastic fluid. The 0.8% (weight/volume) DAG aqueous solution prepared in Experimental Example 1 or a DAG aqueous solution obtained by diluting same to 0.08% (weight/volume) with tap water was mixed with the aforementioned phosphate buffer or microbial nutrient sources-containing aqueous solution to prepare a plastic fluid composed of a phosphate buffer containing 0.04% (weight/volume) DAG (hereinafter a plastic fluid based on a buffer solution such as phosphate buffer is sometimes referred to as "buffer solution-type plastic fluid"), and a plastic fluid composed of a microbial nutrient sources-containing aqueous solution containing 0.04% (weight/volume) DAG (hereinafter a plastic fluid based on a microbial nutrient sources-containing aqueous solution is sometimes referred to as "nutrient source aqueous solution-type plastic fluid"). A three-dimensional medium preparation kit (Nissan Chemical FCeM (registered trademark)-series Preparation Kit) or a stirring vessel (125 mL spinner flask (#3152, manufactured by Corning)) was used to mix the buffer solution and aqueous solution with the DAG aqueous solution according to the following steps.

### <Production of plastic fluid using 3D medium production kit>

In the production of a plastic fluid by using a 3D medium production kit (hereinafter sometimes referred to as "kit method"), 47.5 mL of 1x storage solution was dispensed into a 50 mL conical tube (manufactured by SUMITOMO BAKELITE CO., LTD.) and an adapter cap, which is a component of the kit, was attached. Next, the tip of a disposable syringe filled with 2.5 mL of 0.8% (weight/volume) DAG aqueous solution was connected by fitting same into the cylindrical part of the adapter cap, and the plunger of the syringe was manually pressed to vigorously eject the DAG aqueous solution in the syringe into the container to mix the solution with the storage solution or activation solution, whereby a 0.04% (weight/volume) DAG-containing buffer solution-type plastic fluid or a 0.04% DAG-containing nutrient source aqueous solution-type plastic fluid was prepared.

### <Production of plastic fluid using stirring vessel>

In the production of a plastic fluid by using a stirring vessel (hereinafter sometimes referred to as "stirring mixing method"), 50 mL of 2x concentrated phosphate buffer was added to a 125 mL spinner flask, and 50 mL of 0.08% (weight/volume) DAG aqueous solution was added while stirring at 350 rpm to mix same with the buffer, whereby a 0.04% (weight/volume) DAG-containing buffer solution-type plastic fluid was prepared.

### <Measurement of yield value of plastic fluid>

In addition to each plastic fluid prepared in Experimental Example 2, the yield value was derived as follows for 0.02% (weight/volume), 0.06% (weight/volume), or 0.08% (weight/volume) DAG-containing buffer solution-type or nutrient source aqueous solution-type plastic fluid prepared by two steps as in Experimental Example 2. The shear stress corresponding to each shear rate was measured using a rheometer (manufactured by Anton Paar, model: MCR301, cone rotor: CP75-1). The yield value was derived by calculating the intercept of the linear approximation obtained by plotting the shear rate on the horizontal axis and the shear stress on the vertical axis. The results are shown in Table 1. As shown in Table 1, the liquid not containing DAG did not have a yield value. It was confirmed that the sample group that could maintain microorganisms without sedimentation in a standing state had a yield value.

**[Table 1]**

| yield value (mPa) | Blank | buffer solution-type plastic fluid | | | | nutrient source aqueous solution-type plastic fluid | | | |
|---|---|---|---|---|---|---|---|---|---|
| | 0% | 0.02% | 0.04% | 0.06% | 0.08% | 0.02% | 0.04% | 0.06% | 0.08% |
| kit | - | 33.43 | 72.88 | 186.4 | 276.49 | 8.58 | 22.34 | 37.7 | 42.74 |
| stirring mixing | - | 8.21 | 43.24 | 147 | 270.08 | 8.85 | 25.42 | 42.19 | 59.34 |

### <Measurement of viscosity of plastic fluid>

In addition to the samples prepared in Experimental Example 2, the viscosity was measured as follows for 0.02% (weight/volume), 0.06% (weight/volume), or 0.08% (weight/volume) DAG-containing buffer solution-type plastic fluid or nutrient source aqueous solution-type plastic fluid prepared by two steps as in Experimental Example 2. The viscosity was measured under 4°C or 20°C conditions using an E-type viscometer (manufactured by Toki Sangyo Co., Ltd., TV-22-type viscometer, model: TVE-22 L, cone rotor: standard rotor 1°34'×R24, rotating speed 50 rpm). The results are shown in Table 2. As shown in Table 2, it was confirmed that all plastic fluids had a viscosity that did not interfere with operability, such as filling storage containers.

**[Table 2]**

| viscosity @4°C (mPa·s) | Blank | buffer solution-type plastic fluid | | | | nutrient source aqueous solution-type plastic fluid | | | |
|---|---|---|---|---|---|---|---|---|---|
| | 0% | 0.02% | 0.04% | 0.06% | 0.08% | 0.02% | 0.04% | 0.06% | 0.08% |
| kit | 1. 62 | 5.56 | 11.04 | 23.01 | 41.29 | 2.28 | 2.94 | 3.49 | 4 |
| stirring mixing | 1. 62 | 2.72 | 5.2 | 13.68 | 18.26 | 2.34 | 2.95 | 3.53 | 4.22 |

| viscosity @20°C (mPa·s) | Blank | buffer solution-type plastic fluid | | | | nutrient source aqueous solution-type plastic fluid | | | |
|---|---|---|---|---|---|---|---|---|---|
| | 0% | 0.02% | 0.04% | 0.06% | 0.08% | 0.02% | 0.04% | 0.06% | 0.08% |
| kit | 1.24 | 4.28 | 8.86 | 16.62 | 33.31 | 1.86 | 2.19 | 2.74 | 3.01 |
| stirring mixing | 1.24 | 1. 92 | 3.6 | 10.7 | 13.63 | 1.66 | 2.16 | 2.69 | 3.2 |

### [Experimental Example 3] Production of cellulose fiber (CNF) aqueous dispersion

1000 parts by volume of pure water was added to 1 part by weight of commercially available kraft pulp (LBKPD-8 manufactured by Kokusai Pulp and Paper Co., Ltd., solid content 46% by mass) to disperse the pulp, which was then pulverized 200 times at 245 MPa using a high-pressure pulverizer (Starburst System) manufactured by Sugino Machine Ltd. to obtain an aqueous dispersion of pulp-derived CNF. The obtained dispersion was weighed into a petri dish and dried at 110°C for 1 hr to remove moisture and the solid content of the residue was measured. As a result, the concentration of the CNF solid content in the water was 1.0% (weight/volume).

### [Experimental Example 4] Production of CNF-containing activation solution

Tap water, urea, and various metal salts were mixed to prepare a microbial nutrient sources-containing aqueous solution. The 1.0% (weight/volume) CNF aqueous dispersion produced in Experimental Example 3 was mixed to produce a 0.04% (weight/volume) CNF-containing nutrient source aqueous solution-type plastic fluid. The microbial nutrient sources-containing aqueous solution and the CNF aqueous dispersion were mixed using a three-dimensional medium preparation kit (FCeM (registered trademark)-series Preparation Kit, manufactured by Nissan Chemical Co., Ltd.) according to the following procedure.

### <Production of plastic fluid using 3D medium production kit (kit method)>

48 mL of a microbial nutrient sources-containing aqueous solution was dispensed into a 50 mL conical tube (manufactured by SUMITOMO BAKELITE CO., LTD.) and an adapter cap, which is a component of the kit, was attached. Next, the tip of a disposable syringe filled with 2 mL of 1% (weight/volume) CNF aqueous dispersion was connected by fitting same into the cylindrical part of the adapter cap, and the plunger of the syringe was manually pressed to vigorously eject the CNF aqueous dispersion in the syringe into the container to mix the solution with the microbial nutrient sources-containing aqueous solution, whereby a 0.04% (weight/volume) CNF-containing nutrient source aqueous solution-type plastic fluid was prepared.

### [Experimental Example 5] Coculture of Burkholderia arboris SL1B1 strain (B. arboris) and Yarrowia lipolytica 1A1 strain (Y. lipolytica)

A coculture solution containing fats and oils was placed in the glass tank of a 10L fermenter and sterilized by autoclave. The sterilized coculture solution was placed in the fermenter and a pre-culture solution of B. arboris and a pre-culture solution of Y. lipolytica were inoculated. After inoculation, the fermenter was operated at 30°C for about 28 hr, and then the coculture was terminated.

### [Experimental Example 6] Preparation of standard microbial suspension containing two types of microorganisms

A coculture solution (5 L) was mixed with inorganic salt water containing KH₂PO₄ (600 g) and Na₂HPO₄ (400 g) and adjusted to 100 L to prepare a standard microbial suspension (a 20-fold diluted product of culture solution).

### [Experimental Example 7] Microorganism suspending effect of DAG-containing buffer solution-type plastic fluid

A standard microbial suspension (0.2 L) was dispensed into 6 centrifuge tubes and centrifuged (6000×g, 10 min, 15°C). After centrifugation, the supernatant was discarded, and all of the obtained microbial pellets were suspended in 1 mL of phosphate buffer (KH₂PO₄:6 g/L+Na₂HPO₄:4 g/L) to prepare a 200-fold concentrated standard microbial suspension. Then, 4 ml of phosphate buffer was added to the 200-fold concentrated solution (1 ml) to prepare a 40-fold concentrated standard microbial suspension. Next, 4 ml of phosphate buffer was added to the 40-fold concentrated solution (1 ml) to prepare an 8-fold concentrated standard microbial suspension.

In the storage test using the DAG-containing buffer solution-type plastic fluid, 9.5 mL of 0.04% DAG-containing buffer solution-type plastic fluid ((1) prepared by the kit method, (2) prepared by the stirring mixing method) or (3) 9.5 mL of phosphate buffer not containing DAG was added to a 15 mL tube, and 0.5 mL each of the 200-fold concentrated solution, the 40-fold concentrated solution, and the 8-fold concentrated solution were added and mixed, and then refrigerated to start the test. As the final bacterial cell concentration, a 200-fold concentrated solution is a 10-fold concentrated solution, a 40-fold concentrated solution is a 2-fold concentrated solution, and a 8-fold concentrated solution is a 0.4-fold concentrated solution, based on the standard solution. When roughly calculated based on the dilution standard from the culture solution, the 10-fold concentrated solution corresponds to a 2-fold diluted product, the 2-fold concentrated solution corresponds to a 10-fold diluted product, and the 0.4-fold concentrated solution corresponds to a 50-fold diluted product. The storage period was up to 90 days under refrigeration, and the sedimentation of microorganisms was observed.

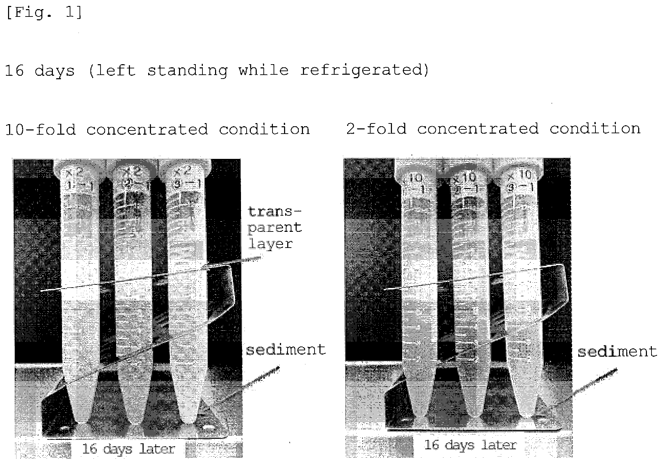

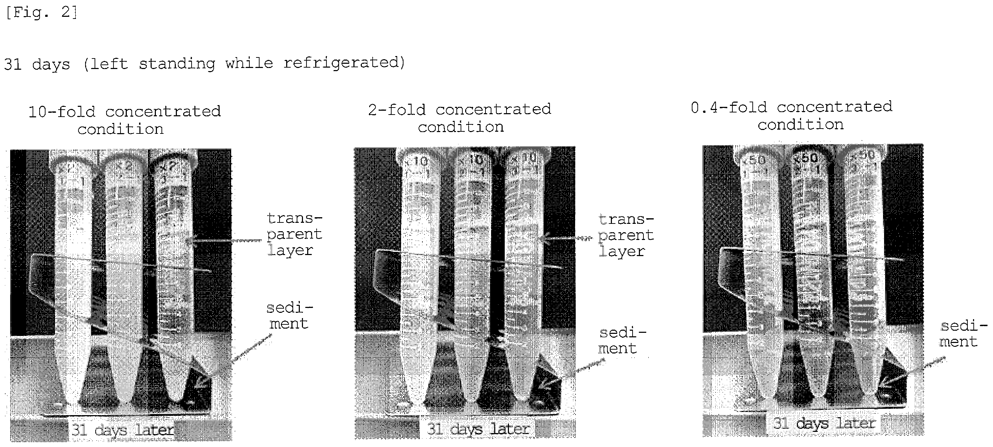

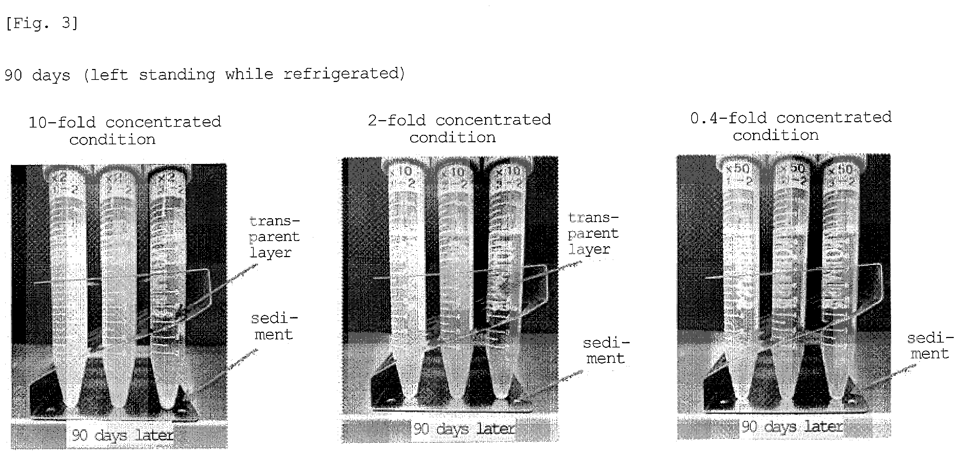

B. arboris and Y. lipolytica were seeded into the DAG-containing buffer solution-type plastic fluid, and photographs showing the suspension state of the microorganisms when stored under refrigerated conditions for 16 days are shown in Fig. 1. In (3) the phosphate buffer not containing DAG, sediment of microorganisms was observed at the bottom in both the 10-fold concentrated solution and the 2-fold concentrated solution. In the 10-fold concentrated solution, the formation of a transparent layer was observed at the top. Furthermore, photographs showing the suspension state of microorganisms after 31 days of storage under refrigerated conditions are shown in Fig. 2, and photographs showing the suspension state of microorganisms after 90 days of storage under refrigerated conditions are shown in Fig. 3. On the 31st day and 90th day, sediment was observed even in the 0.4-fold concentrated solution with (3) phosphate buffer not containing DAG, but no sediment was observed in the DAG-containing buffer solution-type plastic fluid. On the other hand, in the DAG-containing buffer solution-type plastic fluid, neither the sediment nor a transparent layer at the top was observed in either of the preparation methods (1) and (2). The results indicate that the DAG-containing buffer solution-type plastic fluid can suppress microbial sediment even during long-term storage, and can maintain the microorganisms in a uniformly dispersed state in the liquid.

### [Experimental Example 8] Microorganism dispersing effect of DAG-containing buffer solution-type plastic fluid

By a method similar to that in Experimental Example 7, a 200-fold concentrated solution of the standard microbial suspension was prepared.

A storage test using the DAG-containing buffer solution-type plastic fluid was performed as follows. 9.5 mL of 0.04% DAG-containing buffer solution-type plastic fluid ((1) prepared by the kit method, (2) prepared by the stirring mixing method) or (3) 9.5 mL of phosphate buffer not containing DAG was added to a 15 mL tube, and 0.5 mL each of the 200-fold concentrated solution was added thereto (the final bacterial concentration is 10-fold concentration of the standard microbial suspension, and when converted to a dilution standard from the microbial concentration in the microbial culture solution, the "10-fold concentrated standard microbial suspension" corresponds to a 2-fold dilution of the microbial culture solution. After thoroughly mixing each storage solution containing added microorganisms, the solution was left standing under refrigerated conditions. The storage period was 31 days, and sedimentation of the microorganisms was observed on the 31st day.

On the 31st day, in order to confirm the dispersion state of the microorganisms in the storage liquid, 0.5 mL of a sample (supernatant) was collected from the middle part of the container (around 8 mL of the 15 mL tube) of each storage liquid in a standing state. Then, about 9.5 mL of the remaining storage liquid was suspended, and 0.5 mL of the sample after suspending was collected (after suspending). The collected sample was appropriately diluted to a concentration suitable for counting, and 0.1 mL of the diluted sample was collected and dropped onto two plate media, and the bacterial liquid was applied onto the medium by using a conlarger. An oil agar medium was used for colony formation by B. arboris, and an LB agar medium supplemented with an antibiotic was used for colony formation by Y. lipolytica. The plate medium on which the microorganism was applied was cultured at 30°C for three days or more, after which the number of colonies formed on the plate medium was counted and used as the viable cell count. The colony count was the average of the two plate media. The results are shown in Table 3.

**[Table 3]**

| Microorganism dispersibility after refrigerated storage for 31 days | | | | |
|---|---|---|---|---|
| sample | B. arboris | B. arboris | Y. lipolytica | Y. lipolytica |
| | viable cell count (CFU/mL) | viable cell count (CFU/mL) | viable cell count (CFU/mL) | viable cell count (CFU/mL) |
| | supernatant | after suspending | supernatant | after suspending |
| DAG ((1) kit method) | 9.6×10⁸ | 11.0×10⁸ | 16.0×10⁴ | 49.0×10⁴ |
| DAG ((2) stirring mixing method) | 17.0×10⁸ | 12.0×10⁸ | 32.0×10⁴ | 62.0×10⁴ |
| phosphate buffer (3) | 2.4×10⁸ | 18.0×10⁸ | 10 | 38.0×10⁴ |

As shown in Table 3, when the phosphate buffer (3) not containing DAG was used, the viable cell count of B. arboris and Y. lipolytica in the supernatant were low, and most of the microorganisms formed sediments on the bottom surface of the container. On the other hand, when the DAG-containing buffer solution-type plastic fluid was used, many microorganisms were detected in the supernatant in both the (1) kit method and (2) stirring mixing method. The above results indicate that the DAG-containing buffer solution-type plastic fluid can maintain the microorganisms in a dispersed state in the liquid.

### [Experimental Example 9] Microorganism protection effect of DAG-containing buffer solution-type plastic fluid - 1

A protection test using the DAG-containing buffer solution-type plastic fluid was performed as follows. 9.5 mL of 0.04% (weight/volume) DAG-containing buffer solution-type plastic fluid ((1) prepared by the kit method, (2) prepared by the stirring mixing method) or (3) 9.5 mL of phosphate buffer not containing DAG was added to a 15 mL tube, and 0.5 mL each of the 40-fold concentrated solution was added thereto (the final bacterial concentration is 2-fold concentration of the standard microbial suspension, and when converted to a dilution standard from the microbial concentration in the microbial culture solution, the "2-fold concentrated standard microbial suspension" corresponds to a 10-fold dilution product of the microbial culture solution). After thoroughly mixing each storage solution containing added microorganisms, the solution was left standing under refrigerated conditions. The storage period was 31 days, and viable cell counts of the microorganisms (B. arboris and Y. lipolytica) were taken on the 31st day.

On the 31st day, each storage solution was suspended to count the number of microorganisms in the storage solution and appropriately diluted to a concentration suitable for counting, and 0.1 mL of the diluted sample was collected and dropped onto two plate media, and the bacterial liquid was applied onto the medium by using a conlarger. An oil agar medium was used for colony formation by B. arboris, and an LB agar medium supplemented with an antibiotic was used for colony formation by Y. lipolytica. The plate medium on which the microorganism was applied was cultured at 30°C for three days or more, after which the number of colonies formed on the plate medium was counted and used as the viable cell count. The colony count was the average of the two plate media. The results are shown in Table 4.

**[Table 4]**

| Each viable count after refrigerated storage for 31 days (2-fold concentrated solution condition) | | |
|---|---|---|
| sample | B. arboris | Y. lipolytica |
| | viable cell count (CFU/mL) | viable cell count (CFU/mL) |
| DAG ((1) kit method) | 4.9×10⁸ | 7.0×10⁴ |
| DAG ((2) stirring mixing method) | 5.0×10⁸ | 7.4×10⁴ |
| phosphate buffer (3) | 3.2×10⁸ | 6.0×10⁴ |
| at start of storage (day 0) | 6.3×10⁸ | 7.4×10⁴ |

As shown in Table 4, the DAG-containing buffer solution-type plastic fluid showed a higher microorganism protection effect than the phosphate buffer not containing DAG. A comparison of the viable cell counts between the start of storage and after storage also revealed that the DAG-containing buffer solution-type plastic fluid has a higher microorganism protection effect than the phosphate buffer not containing DAG.

### [Experimental Example 10] Microorganism protection effect of DAG-containing buffer solution-type plastic fluid - 2 (high concentration and long period of time)

A protection test using the DAG-containing buffer solution-type plastic fluid was performed as follows. 9.5 mL of 0.04% (weight/volume) DAG-containing buffer solution-type plastic fluid ((1) prepared by the kit method, (2) prepared by the stirring mixing method) or (3) 9.5 mL of phosphate buffer not containing DAG was added to a 15 mL tube, and 0.5 mL each of the 200-fold concentrated solution was added thereto (the final bacterial concentration is 10-fold concentration of the standard microbial suspension, and when converted to a dilution standard from the microbial concentration in the microbial culture solution, the "10-fold concentrated standard microbial suspension" corresponds to 2-fold dilution of the microbial culture solution). After thoroughly mixing each storage solution containing added microorganisms, the solution was left standing under refrigerated conditions. The storage period was 90 days, and viable cell counts of the microorganisms (B. arboris and Y. lipolytica) were taken on the 90th day.

On the 90th day, each storage solution was suspended to count the number of microorganisms in the storage solution and appropriately diluted to a concentration suitable for counting, and 0.1 mL of the diluted sample was collected and dropped onto two plate media, and the bacterial liquid was applied onto the medium by using a conlarger. An oil agar medium was used for colony formation by B. arboris, and an LB agar medium supplemented with an antibiotic was used for colony formation by Y. lipolytica. The plate medium on which the microorganism was applied was cultured at 30°C for three days or more, after which the number of colonies formed on the plate medium was counted and used as the viable cell count. The colony count was the average of the two plate media. The results are shown in Table 5.

**[Table 5]**

| Each viable count after refrigerated storage for 90 days | | |
|---|---|---|
| sample | B. arboris viable cell count (CFU/mL) | Y. lipolytica viable cell count (CFU/mL) |
| buffer solution-type plastic fluid ((1) kit method) | 2.3×10⁸ | 15.0×10⁴ |
| buffer solution-type plastic fluid ((2) stirring mixing method) | 1.7×10⁸ | 9.2×10⁴ |
| phosphate buffer (3) | 0.8×10⁸ | 5.1×10⁴ |

As shown in Table 5, the DAG-containing buffer solution-type plastic fluid showed a higher microorganism protection effect than the phosphate buffer not containing DAG even under the conditions of high concentration and long period of time.

### [Experimental Example 11] Effect of DAG-containing buffer solution-type plastic fluid on suppressing decline of microbial proliferation function

After the bacterial count in Experimental Example 8, the DAG-containing buffer solution-type plastic fluid ((1) prepared by the kit method) and the phosphate buffer not containing DAG (3) were further stored under refrigeration for about 2 weeks (about 3.5 months from the start of storage). Thereafter, each sample was appropriately diluted with a phosphate buffer not containing DAG to a concentration suitable for counting, and the diluted sample was re-cultured in a system using a compact culture device (1.6 L) and simulated wastewater to confirm the proliferation function of microorganisms. The composition of the simulated wastewater was as follows:
urea 45 mg/L
KH₂PO₄ 5.25 mg/L
Na₂HPO₄ 1.41 mg/L
canola oil 500 ppm/L
Triton-X 50 ppm/L

Cultivation in the compact culture device was performed for 24 hr under conditions of constant stirring at 240 rpm at 30°C. Sampling for counting the number of bacteria was performed at the start, 16 hr and 24 hr.

The number of bacteria of the microorganisms cultured in the simulated wastewater was counted according to the following procedure. The samples after culture were suspended well. Each sample was appropriately diluted to a concentration suitable for counting, and 0.1 mL of the diluted sample was collected and dropped onto two plate media, and the bacterial liquid was applied onto the medium by using a conlarger. An oil agar medium was used for colony formation by B. arboris, and an LB agar medium supplemented with an antibiotic was used for colony formation by Y. lipolytica. The plate medium on which the microorganism was applied was cultured at 30°C for three days or more, after which the number of colonies formed on the plate medium was counted and used as the viable cell count. The colony count was the average of the two plate media. The results are shown in Table 6 (B. arboris) and Table 7 (Y. lipolytica).

**[Table 6]**

| B. arboris viable cell count in simulated wastewater culture after refrigerated storage for about 3.5 months | | | |
|---|---|---|---|
| B. arboris viable cell count (CFU/mL) | before culture | 16 hr | 24 hr |
| buffer solution-type plastic fluid ((1) kit method) | 7.4×10⁵ | 110×10⁵ | 440×10⁵ |
| phosphate buffer (3) | 4.2×10⁵ | 5.0×10⁵ | 1200×10⁵ |

**[Table 7]**

| Y. lipolytica viable cell count in simulated wastewater culture after refrigerated storage for about 3.5 months | | | |
|---|---|---|---|
| Y. lipolytica viable cell count (CFU/mL) | before culture | 16 hr | 24 hr |
| buffer solution-type plastic fluid ((1) kit method) | 2.2×10² | 10.0×10² | 18.0×10² |
| phosphate buffer (3) | 0.85×10² | 7.0×10² | 0.1×10² |

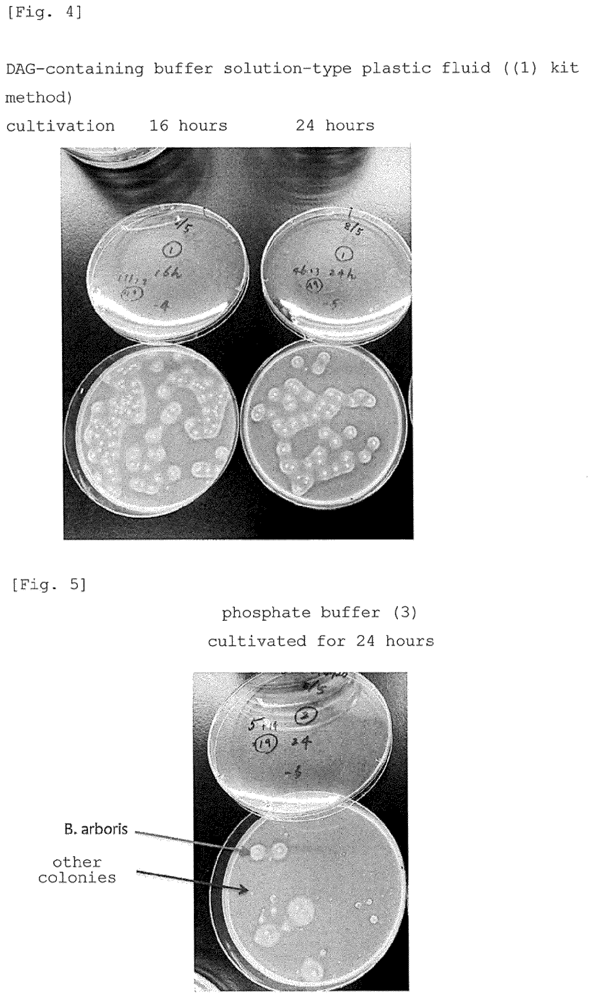

As shown in Table 6 and Table 7, B. arboris and Y. lipolytica stored for about 3.5 months using DAG-containing buffer solution-type plastic fluid showed good proliferation under simulated wastewater. On the other hand, B. arboris and Y. lipolytica stored for about 3.5 months using phosphate buffer not containing DAG showed slow proliferation. In particular, the viable cell count of Y. lipolytica stored for about 3.5 months using phosphate buffer not containing DAG dropped sharply at 24 hr. Interestingly, when the DAG-containing buffer solution-type plastic fluid was used, only B. arboris proliferated (Fig. 4), whereas when the phosphate buffer not containing DAG was used, many colonies considered to be derived from microorganisms other than B. arboris were confirmed (Fig. 5). These results demonstrate that the DAG-containing buffer solution-type plastic fluid has the effect of suppressing the decline in the proliferation function of stored microorganisms, and also has the effect of suppressing the proliferation of microorganisms other than the desired microorganisms.

### [Experimental Example 12] Effect of DAG-containing nutrient source aqueous solution-type plastic fluid on suppressing decline of microbial proliferation function

A storage test using a DAG-containing nutrient source aqueous solution-type plastic fluid was performed as follows: 9.5 mL of a 0.04% (weight/volume) DAG-containing nutrient source aqueous solution-type plastic fluid ((1) kit method) or 9.5 mL of a DAG-free microbial nutrient sources-containing aqueous solution (3) was each added to a 15 mL tube, 0.5 mL of a 200-fold concentrated solution was added thereto (final bacterial cell concentration is 10-fold concentration of the standard microbial suspension, and when converted to a dilution standard based on the microbial concentration in the microbial culture solution, a "10-fold concentration of the standard microbial suspension" corresponds to a 2-fold dilution of the microbial culture solution). The storage period was about 1.5 months under refrigeration.

After storage, each sample was appropriately diluted with a DAG-free microbial nutrient sources-containing aqueous solution to a concentration suitable for counting. A microbial nutrient sources-containing aqueous solution and canola oil were added to the diluted sample, and the sample was re-cultured using a compact culture device (1.6 L). Cultivation was performed continuously for 64 hr under conditions of constant stirring at 240 rpm at 30°C. Photographing of the culture state was performed at the start, 24 hr, 48 hr, and 64 hr. Sampling for counting the number of bacteria was performed at the start, 40 hr and 64 hr.

The number of bacteria of the microorganisms cultured using a compact culture device was counted according to the following procedure. The samples after culture were suspended well. Each sample was appropriately diluted to a concentration suitable for counting, and 0.1 mL of the diluted sample was collected and dropped onto two plate media, and the bacterial liquid was applied onto the medium by using a conlarger. An oil agar medium was used for colony formation by B. arboris, and an LB agar medium supplemented with an antibiotic was used for colony formation by Y. lipolytica. The plate medium on which the microorganism was applied was cultured at 30°C for three days or more, after which the number of colonies formed on the plate medium was counted and used as the viable cell count. The colony count was the average of the two plate media. The results are shown in Table 8 (B. arboris) and Table 9 (Y. lipolytica).

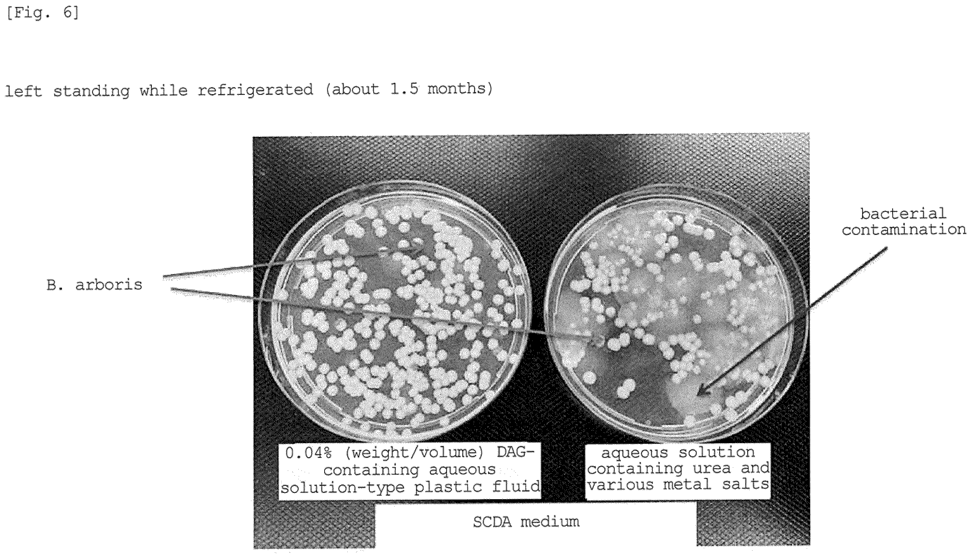

For counting the number of general bacteria including B. arboris by re-culture, each sample at the time point of 64 hr was appropriately diluted to a concentration suitable for counting, 0.1 mL was collected and dropped onto a SCDA plate medium (Tryptic Soy Agar; manufactured by Merck Millipore), and the bacterial liquid was applied onto the medium by using a conlarger. The sample was cultured at 30°C for 3 days or more, and then photographed to determine the presence or absence of contamination with other bacteria. The results are shown in Fig. 6.

**[Table 8]**

| B. arboris viable cell count in re-culture after refrigerated storage for about 1.5 months | | | |
|---|---|---|---|
| B. arboris viable cell count (CFU/mL) | before culture | 40 hr | 64 hr |
| DAG-containing nutrient source aqueous solution-type plastic fluid ((1) kit method) | 1.8×10⁶ | 620×10⁶ | 5000×10⁶ |
| microbial nutrient sources-containing aqueous solution (3) | 1.5×10⁶ | 290×10⁶ | 2500×10⁶ |

**[Table 9]**

| Y. lipolytica viable cell count in re-culture after refrigerated storage for about 1.5 months | | | |
|---|---|---|---|
| Y. lipolytica viable cell count (CFU/mL) | before culture | 40 hr | 64 hr |
| DAG-containing nutrient source aqueous solution-type plastic fluid ((1) kit method | 8.0×10² | 40×10² | 300×10² |
| microbial nutrient sources-containing aqueous solution (3) | 7.4×10² | 25×10² | 160×10² |

As shown in Table 8 and Table 9, B. arboris and Y. lipolytica stored for about 1.5 months using DAG-containing nutrient source aqueous solution-type plastic fluid showed good proliferation under re-culture. On the other hand, B. arboris and Y. lipolytica stored for about 1.5 months using DAG-free microbial nutrient sources-containing aqueous solution also showed proliferation in re-culture; however, the proliferation rate thereof was lower than the conditions using a DAG-containing nutrient source aqueous solution-type plastic fluid.

In addition, the SCDA method was performed to confirm the presence or absence of contamination with other bacteria in the culture solution at 64 hr after the start of re-culture. As in Experimental Example 11, when the DAG-containing nutrient source aqueous solution-type plastic fluid was used, B. arboris mainly proliferated, whereas when a DAG-free microbial nutrient sources-containing aqueous solution was used, many colonies considered to be derived from microorganisms other than B. arboris were confirmed (Fig. 6). These results demonstrate that the DAG-containing nutrient source aqueous solution-type plastic fluid has the effect of suppressing the decline in the proliferation function of stored microorganisms, and also has the effect of suppressing the proliferation of microorganisms other than the desired microorganisms.

### [Experimental Example 13] Effect of DAG-containing nutrient source aqueous solution-type plastic fluid on suppressing microbial alteration and decline of microbial proliferation function

A storage test using a DAG-containing nutrient source aqueous solution-type plastic fluid was performed as follows: 9.5 mL of a 0.04% DAG-containing nutrient source aqueous solution-type plastic fluid ((1) kit method) or 9.5 mL of a DAG-free microbial nutrient sources-containing aqueous solution (2) was each added to a 15 mL tube, 0.5 mL of a pre-culture solution of B. arboris cultivated under a clean bench was added to an initial density of 10⁸ CFU/mL or more. Each sample was mixed well and stored under refrigeration conditions. The storage period was set to 28 days.

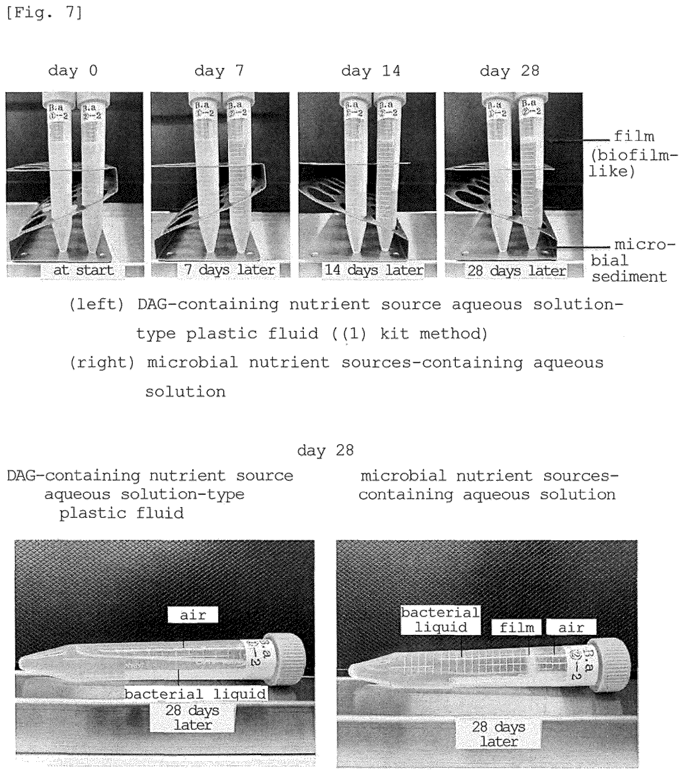

In a sample stored using a DAG-free microbial nutrient sources-containing aqueous solution, a biofilm-like film was formed over time. It was speculated that B. arboris was altered during refrigerated storage and formed a biofilm (Fig. 7). On the other hand, in the sample stored using a DAG-containing nutrient source aqueous solution-type plastic fluid, biofilm formation by B. arboris was not observed even after passage of time (Fig. 7). By the present experiment, it was shown that a DAG-containing nutrient source aqueous solution-type plastic fluid has an effect of suppressing microbial alteration.

The refrigerated storage was further continued to 43 days and each sample was recovered on the 43rd day. A DAG-free microbial nutrient sources-containing aqueous solution and canola oil were added to the recovered sample, and the sample was re-cultured using a compact culture device (1.6 L). Cultivation was performed under conditions of constant stirring at 240 rpm at 30°C. The cultivation was performed for 24 hr. Sampling for counting the number of bacteria was performed at the start, 16 hr and 24 hr of re-culture.

The number of bacteria in the sample of each storage and the sample of re-culture was counted according to the following procedure. Each sample was suspended well. Each sample was appropriately diluted to a concentration suitable for counting, and 0.1 mL thereof was collected and dropped onto two plate media, and the bacterial liquid was applied onto the medium by using a conlarger. An oil agar medium was used for colony formation by B. arboris. The plate medium on which the microorganism was applied was cultured at 30°C for three days or more, after which the number of colonies formed on the plate medium was counted and used as the viable cell count. The colony count was the average of the two plate media. The results are shown in Table 10.

**[Table 10]**

| B. arboris proliferability (CFU/mL) by re-culture after refrigerated storage for 43 days | | |
|---|---|---|
| when and time | DAG-containing nutrient source aqueous solution-type plastic fluid ((1) kit method) | microbial nutrient sources-containing aqueous solution (2) |
| before start | 0.1×10⁶ | 0.022×10⁶ |
| 16 hr | 2.0×10⁶ | 0.043×10⁶ |
| 24 hr | 4200×10⁶ | 2.5×10⁶ |

As shown in Table 10, B. arboris stored using DAG-containing nutrient source aqueous solution-type plastic fluid showed good proliferation under re-culture. On the other hand, B. arboris stored using DAG-free microbial nutrient sources-containing aqueous solution also showed proliferation in re-culture; however, the proliferation rate thereof was lower than the conditions using a DAG-containing nutrient source aqueous solution-type plastic fluid.

### [Experimental Example 14] Microorganism dispersing effect of CNF-containing nutrient source aqueous solution-type plastic fluid

A standard microbial suspension (0.2 L) was dispensed into 4 centrifuge tubes and centrifuged (6000×g, 10 min, 15°C). After centrifugation, the supernatant was discarded, and all of the obtained microbial pellets were suspended in phosphate buffer (KH₂PO₄:6 g/L+Na₂HPO₄:4 g/L) to prepare a 200-fold concentrated standard microbial suspension.

A storage test using the DAG-containing nutrient source aqueous solution-type plastic fluid or CNF-containing nutrient source aqueous solution-type plastic fluid was performed as follows. (1) 9.5 mL of a microbial nutrient sources-containing aqueous solution and not containing DAG or CNF, (2) 9.5 mL of a 0.04% (weight/volume) CNF-containing nutrient source aqueous solution-type plastic fluid, or (3) 9.5 mL of a 0.04% (weight/volume) DAG-containing nutrient source aqueous solution-type plastic fluid was added to a 15 mL tube, and 0.5 mL each of the 200-fold concentrated solution was added thereto (the final bacterial concentration is 10-fold concentration of the standard microbial suspension, and when converted to a dilution standard from the microbial concentration in the microbial culture solution, the "10-fold concentrated standard microbial suspension" corresponds to a 2-fold dilution of the microbial culture solution). After thoroughly mixing each storage solution containing added microorganisms, the solution was left standing under refrigerated conditions. The storage period was 31 days, and sedimentation of the microorganisms was observed on the 31st day.

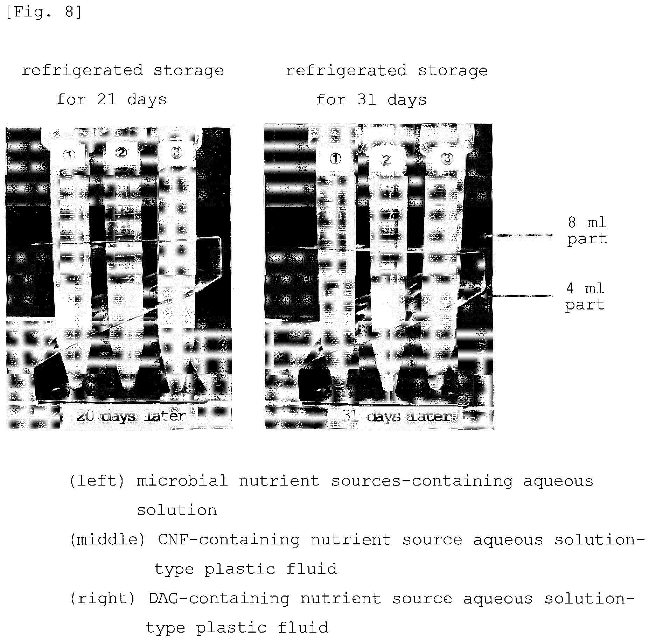

On the 31st day, in order to confirm the dispersion state of the microorganisms in the activation liquid, 0.5 mL of a sample (supernatant) was collected from the middle part of the container (around 8 mL of the 15 mL tube) of each storage liquid in a standing state. The collected sample was appropriately diluted to a concentration suitable for counting, and 0.1 mL of the diluted sample was collected and dropped onto two plate media, and the bacterial liquid was applied onto the medium by using a conlarger. An oil agar medium was used for colony formation by B. arboris, and an LB agar medium supplemented with an antibiotic was used for colony formation by Y. lipolytica. The plate medium on which the microorganism was applied was cultured at 30°C for three days or more, after which the number of colonies formed on the plate medium was counted and used as the viable cell count. The colony count was the average of the two plate media. The results are shown in Fig. 8 (photographs at the time points of 21st day and 31st day), Table 11 (B. arboris) and Table 12 (Y. lipolytica).

**[Table 11]**

| Microorganism dispersibility (B. arboris; CFU/mL) after refrigerated storage for 31 days | | | |
|---|---|---|---|
| sample | microbial nutrient sources-containing aqueous solution | CNF-containing nutrient source aqueous solution-type plastic fluid | DAG-containing nutrient source aqueous solution-type plastic fluid |
| supernatant 8 mL | 140×10⁶ | 35×10⁶ | 3800×106 |
| supernatant 4 mL | 110×10⁶ | 410×10⁶ | 3800×10⁶ |

**[Table 12]**

| Microorganism dispersibility (Y. lipolytica; CFU/mL) after refrigerated storage for 31 days | | | |
|---|---|---|---|
| sample | microbial nutrient sources-containing aqueous solution | CNF-containing nutrient source aqueous solution-type plastic fluid | DAG-containing nutrient source aqueous solution-type plastic fluid |
| supernatant 8 mL | 0.025×10³ | < 0.01×10³ | 4000×10³ |
| supernatant 4 mL | 0.04×10³ | 2.4×10³ | 1600×10³ |

As shown in Fig. 8, the (1) microbial nutrient sources-containing aqueous solution not containing DAG or CNF and (2) CNF-containing nutrient source aqueous solution-type plastic fluid showed a transparent supernatant from the 21st day. As shown in Table 11 and Table 12, the viable cell counts of B. arboris and Y. lipolytica in these supernatants were lower than those of the supernatant of the DAG-containing nutrient source aqueous solution-type plastic fluid. From the above results, it was shown that the DAG-containing nutrient source aqueous solution-type plastic fluid has a more preferred suspending and dispersing effect as compared with the CNF-containing nutrient source aqueous solution-type plastic fluid.

### [Experimental Example 15] Microorganism protection effect of DAG- or CNF-containing nutrient source aqueous solution-type plastic fluid

A standard microbial suspension (0.2 L) was dispensed into 4 centrifuge tubes and centrifuged (6000×g, 10 min, 15°C). After centrifugation, the supernatant was discarded, and all of the obtained microbial pellets were suspended in phosphate buffer (KH₂PO₄:6 g/L+Na₂HPO₄:4 g/L) to prepare a 200-fold concentrated standard microbial suspension.

A storage test using the DAG-containing nutrient source aqueous solution-type plastic fluid or CNF-containing nutrient source aqueous solution-type plastic fluid was performed as follows. (1) 9.5 mL of a microbial nutrient sources-containing aqueous solution and not containing DAG or CNF, (2) 9.5 mL of a 0.04% (weight/volume) CNF-containing nutrient source aqueous solution-type plastic fluid, or (3) 9.5 mL of a 0.04% (weight/volume) DAG-containing nutrient source aqueous solution-type plastic fluid was added to a 15 mL tube, and 0.5 mL each of the 200-fold concentrated solution was added thereto (the final bacterial concentration is 10-fold concentration of the standard microbial suspension, and when converted to a dilution standard from the microbial concentration in the microbial culture solution, the "10-fold concentrated standard microbial suspension" corresponds to a 2-fold dilution of the microbial culture solution. After thoroughly mixing each storage solution containing added microorganisms, the solution was left standing under refrigerated conditions). The storage period was 95 days, and the numbers of viable microorganisms (B. arboris and Y. lipolytica) were counted on the 95th day.

On the 95th day, each storage solution was suspended to count the number of microorganisms in the storage solution and appropriately diluted to a concentration suitable for counting, and 0.1 mL of the diluted sample was collected and dropped onto two plate media, and the bacterial liquid was applied onto the medium by using a conlarger. An oil agar medium was used for colony formation by B. arboris, and an LB agar medium supplemented with an antibiotic was used for colony formation by Y. lipolytica. The plate medium on which the microorganism was applied was cultured at 30°C for three days or more, after which the number of colonies formed on the plate medium was counted and used as the viable cell count. The colony count was the average of the two plate media. The results are shown in Table 13 and Table 14.

**[Table 13]**

| Microorganism dispersibility (B. arboris; CFU/mL) after refrigerated storage for 95 days | | | |
|---|---|---|---|
| sample | microbial nutrient sources-containing aqueous solution | CNF-containing nutrient source aqueous solution-type plastic fluid | DAG-containing nutrient source aqueous solution-type plastic fluid |
| B. arboris number | 270×10⁶ | 680×10⁶ | 1600×10⁶ |

**[Table 14]**

| Microorganism dispersibility (Y. lipolytica; CFU/mL) after refrigerated storage for 95 days | | | |
|---|---|---|---|
| sample | microbial nutrient sources-containing aqueous solution | CNF-containing nutrient source aqueous solution-type plastic fluid | DAG-containing nutrient source aqueous solution-type plastic fluid |
| Y. lipolytica number | 270×10³ | 290×10³ | 1500×10³ |

As shown in Table 13 and Table 14, the DAG-containing nutrient source aqueous solution-type plastic fluid and the CNF-containing nutrient source aqueous solution-type plastic fluid showed a microorganism protection effect under the conditions of high concentration and long period of time.

### [Experimental Example 16] Microorganism protection effect of DAG-containing nutrient source aqueous solution-type plastic fluid - 3 (imparting resistance to high temperature and alkali)

A standard microbial suspension (0.1 L) containing Y. lipolytica was dispensed into centrifuge tubes and centrifuged (6000×g, 10 min, 15°C). After centrifugation, the supernatant was discarded, and all of the obtained microbial pellets were suspended in 2.5 mL of a microbial nutrient sources-containing aqueous solution to prepare a 40-fold concentrated standard microbial suspension.

A storage test using a DAG-containing nutrient source aqueous solution-type plastic fluid was performed as follows: 9.5 mL of a 0.04% (weight/volume) DAG-containing nutrient source aqueous solution-type plastic fluid (kit method) or 9.5 mL of a DAG-free microbial nutrient sources-containing aqueous solution was each added to a 15 mL tube, 0.5 mL of a 40-fold concentrated solution was added thereto (final bacterial cell concentration is 2-fold concentration of the standard microbial suspension, and when converted to a dilution standard based on the microbial concentration in the microbial culture solution, a "2-fold concentration of the standard microbial suspension" corresponds to a 10-fold dilution of the microbial culture solution). Two samples of the microorganism-added DAG-containing nutrient source aqueous solution-type plastic fluid and two samples of the microorganism-added DAG-free microbial nutrient sources-containing aqueous solution were prepared. The storage period was 14 days under refrigeration.

At 14 days of refrigerated storage, 3.2 mL of each sample was collected and re-cultured in a system using a compact culture device (1.6 L) and simulated wastewater to confirm the proliferation function of microorganisms. The composition of the simulated wastewater was as follows:

urea 45 mg/L
KH₂PO₄ 5.25 mg/L
Na₂HPO₄ 1.41 mg/L
canola oil 500 ppm/L
Triton-X 50 ppm/L

Cultivation in the compact culture device was performed under the following two conditions:
[condition 1] constant stirring at 40°C, pH 7.3, 120 rpm, cultured for 6 hr
[condition 2] constant stirring at 30°C, pH10, 120 rpm, cultured for 6 hr

Sampling to count the number of bacteria was performed at the start of cultivation and 6 hr after the start of cultivation.

The number of bacteria of the microorganisms cultured in the simulated wastewater was counted as follows. Each sample after culture was suspended, appropriately diluted to a concentration suitable for counting, and 0.1 mL of the diluted sample was collected and dropped onto two plate media, and the bacterial liquid was applied onto the medium by using a conlarger. An LB agar supplemented with an antibiotic was used as a plate medium. The plate medium on which the microorganism was applied was cultured at 30°C for three days or more, after which the number of colonies formed on the plate medium was counted and used as the viable cell count. The colony count was the average of the two plate media. The results of culturing the microorganisms under condition 1 are shown in Table 15, and the results of culturing the microorganisms under condition 2 are shown in Table 16.

**[Table 15]**

| Viable cell count (CFU/mL) of Y. lipolytica under 40°C culture conditions | | |
|---|---|---|
| | before culture | 6 hr |
| DAG-containing nutrient source aqueous solution-type plastic fluid (kit method) | 920 | 75 |
| microbial nutrient sources-containing aqueous solution | 740 | <10 |

**[Table 16]**

| Viable cell count (CFU/mL) of Y. lipolytica under pH10 culture conditions | | |
|---|---|---|
| Y. lipolytica viable cell maintaining effect (%) (comparison with before culture) | before culture | 6 hr |
| DAG-containing nutrient source aqueous solution-type plastic fluid (kit method) | 920 | 1400 |
| microbial nutrient sources-containing aqueous solution | 740 | < 10 |

As shown in Table 15 and Table 16, it was shown that Y. lipolytica stored using a plastic fluid suppressed a decrease in the survival rate under high temperature conditions and alkali conditions as compared with Y. lipolytica stored without using a plastic fluid.

From the above results, it was shown that the storage of microorganism in a DAG-containing nutrient source aqueous solution-type plastic fluid can impart resistance to high temperature and alkali.

### [Experimental Example 17] Fats and oils decomposition effect of microbial liquid containing DAG-containing buffer solution-type plastic fluid

In the storage test using the DAG-containing buffer solution-type plastic fluid, 9 mL of 0.04% DAG-containing buffer solution-type plastic fluid ((2) prepared by the stirring mixing method) or (3) 9 mL of phosphate buffer not containing DAG was added to a 15 mL tube, and 1 mL of a microbial culture solution was added and mixed to give a 10-fold diluted product, which was then refrigerated to start the test. The storage period was 43 days under refrigeration, and the sedimentation of microorganisms was observed.

At 43 days of refrigerated storage, the sample was suspended in a storage solution, and 0.5 mL of the suspended sample was collected (after suspending). The collected sample was appropriately diluted to a concentration suitable for counting, and 0.1 mL of the diluted sample was collected and dropped onto two plate media, and the bacterial liquid was applied onto the medium by using a conlarger. An oil agar medium was used for colony formation by B. arboris, and an LB agar medium supplemented with an antibiotic was used for colony formation by Y. lipolytica. The plate medium on which the microorganism was applied was cultured at 30°C for three days or more, after which the number of colonies formed on the plate medium was counted and used as the viable cell count. The colony count was the average of the two plate media.

Furthermore, 1 mL of each sample was collected and re-cultured in a system using a flask (100 mL) and simulated wastewater to confirm the proliferation function and fats and oils decomposability of microorganisms. The flask culture was performed at 30°C, 90 rpm for 64 hr. The fats and oils decomposability was confirmed by measuring the mass of normal hexane extract, which is a conventional method. The proliferation function of the microorganisms was measured using the above-mentioned bacteria count method.

The composition of the simulated wastewater was as follows:
urea 45 mg/L
KH₂PO₄ 5.25 mg/L
Na₂HPO₄ 1.41 mg/L
canola oil 500 ppm/L
Triton-X 50 ppm/L

The proliferation function of B. arboris in simulated wastewater is shown in Table 17, the proliferation function of Y. lipolytica in simulated wastewater is shown in Table 18, and fats and oils decomposability is shown in Table 19.

**[Table 17]**

| B. arboris viable cell count in simulated wastewater culture after refrigerated storage for 43 days | | | |
|---|---|---|---|
| B. arboris viable cell count (CFU/mL) | before culture | 64 hr | proliferation ratio (64 hr/before culture) |
| DAG ((2) stirring mixing method) | 2.5 x 10⁶ | 39 x 10⁶ | 15.6 |
| phosphate buffer (3) | 1.4 x 10⁶ | 5 x 10⁶ | 3.6 |

**[Table 18]**

| Y. lipolytica viable cell count in simulated wastewater culture after refrigerated storage for 43 days | | | |
|---|---|---|---|
| Y. lipolytica viable cell count (CFU/mL) | before culture | 64 hr | proliferation ratio (64 hr/before culture) |
| DAG ((2) stirring mixing method) | 150 | 270 | 1.8 |
| phosphate buffer (3) | 250 | 120 | 0.5 |

**[Table 19]**

| Fats and oils decomposability in simulated wastewater culture after refrigerated storage for 43 days | | |
|---|---|---|
| fats and oils decomposability normal hexane extract (mg/L) | before culture | 64 hr |
| DAG ((2) stirring mixing method) | 493 | 339 |
| phosphate buffer (3) | 493 | 384 |

From the above results, B. arboris and Y. lipolytica stored for a long time using a DAG-containing buffer solution-type plastic fluid had higher proliferability than those stored for a long time using a buffer not containing DAG. In addition, B. arboris and Y. lipolytica stored for a long time using a DAG-containing buffer solution-type plastic fluid maintained fats and oils decomposability. From the above, it was shown that storage of microorganisms using a DAG-containing buffer solution-type plastic fluid can maintain not only the proliferability of the microorganisms but also fats and oils decomposability thereof.

### [Industrial Applicability]

According to the present invention, one type or multiple types of microorganisms can be stored in a liquid state at high concentrations for a long period of time without accompanying functional deterioration, while suppressing contamination with other microorganisms. According to the present invention, moreover, resistance to high temperature and alkali can be imparted to microorganisms. Therefore, the present invention is beneficial for, for example, production of microbial preparations.

This application is based on a patent application No. 2022-096055 filed in Japan (filing date: June 14, 2022) and a patent application No. 2023-008889 filed in Japan (filing date: January 24, 2023), the contents of which are incorporated in full herein.

## Claims

1. A plastic fluid composition comprising the following:
(1) a microorganism
(2) a polysaccharide or a nanofiber composed of a polysaccharide,
wherein when the microorganism is a bacterium, it is comprised at a microbial density of 1×10⁷ CFU/mL or more, and is dispersed in the composition, and when the microorganism is a fungi, it is comprised at a microbial density of 1×10³ CFU/mL or more, and is dispersed in the composition.

2. The composition according to claim 1, wherein the composition has a yield value of 5 to 500 mPa and a viscosity of 1.5 to 200 mPa·s at 4 to 25°C.

3. The composition according to claim 1 or 2, comprising a polysaccharide.

4. The composition according to claim 3, wherein the polysaccharide is a deacylated gellan gum.

5. The composition according to claim 1 or 2, comprising a nanofiber composed of a polysaccharide.

6. The composition according to claim 5, wherein the nanofiber is a cellulose nanofiber.

7. The composition according to claim 1 or 2, which is for storing a microorganism.

8. The composition according to claim 1 or 2, comprising two types of microorganisms.

9. The composition according to claim 8, wherein the two types of microorganisms are a bacterium and a fungi.

10. The composition according to claim 9, wherein the bacterium is Burkholderia arboris and the fungi is Yarrowia lipolytica.

11. A method for producing a microorganism-containing preparation, comprising dispersing a microorganism in a plastic fluid composition comprising a polysaccharide or a nanofiber composed of a polysaccharide,
wherein when the microorganism is a bacterium, it is comprised at a microbial density of 1×10⁷ CFU/mL or more, and is dispersed in the composition, and when the microorganism is a fungi, it is comprised at a microbial density of 1×10³ CFU/mL or more, and is dispersed in the composition.

12. The method according to claim 11, wherein the plastic fluid has the following characteristics:
having a yield value of 5 to 500 mPa and a viscosity of 1.5 to 200 mPa·s at 4 to 25°C.

13. The method according to claim 11 or 12, wherein the composition comprises a polysaccharide.

14. The method according to claim 13, wherein the polysaccharide is a deacylated gellan gum.

15. The method according to claim 11 or 12, wherein the composition comprises a nanofiber composed of a polysaccharide.

16. The method according to claim 15, wherein the nanofiber is a cellulose nanofiber.

17. The method according to claim 11 or 12, comprising two types of microorganisms.

18. The method according to claim 17, wherein the two types of microorganisms are a bacterium and a fungi.

19. The method according to claim 18, wherein the bacterium is Burkholderia arboris and the fungi is Yarrowia lipolytica.
